(19) Europäisches Patentamt
European Patent Office
Office européen des brevets

(11) Veröffentlichungsnummer: **0 539 713 A1**

# (12) EUROPÄISCHE PATENTANMELDUNG

(21) Anmeldenummer: **92115969.5**

(22) Anmeldetag: **18.09.92**

(51) Int. Cl.5: **C07D 403/10**, A61K 31/415, C07D 409/14, A61K 31/41, A61K 31/38

(30) Priorität: **01.10.91 DE 4132632**

(43) Veröffentlichungstag der Anmeldung: **05.05.93 Patentblatt 93/18**

(84) Benannte Vertragsstaaten: **AT BE CH DE DK ES FR GB GR IE IT LI LU MC NL PT SE**

(71) Anmelder: **BAYER AG**

**W-5090 Leverkusen 1 Bayerwerk(DE)**

(72) Erfinder: **Krämer, Thomas, Dr.**
**In den Birken 92a**
**W-5600 Wuppertal 1(DE)**
Erfinder: **Dressel, Jürgen, Ph. D.**
**Claudiusweg 9**
**W-5600 Wuppertal 1(DE)**
Erfinder: **Hanko, Rudolf, Dr.**
**Waldsaum 25**
**W-4300 Essen(DE)**
Erfinder: **Hübsch, Walter, Dr.**
**Wildsteig 22**
**W-5600 Wuppertal 1(DE)**
Erfinder: **Müller, Ulrich, Dr.**
**Claudiusweg 5**
**W-5600 Wuppertal 1(DE)**
Erfinder: **Müller-Gliemann, Matthias, Dr.**
**Claudiusweg 5**
**W-5600 Wuppertal 1(DE)**
Erfinder: **Beuck, Martin, Dr.**
**Trills 7**
**W-4006 Erkrath 2(DE)**
Erfinder: **Kazda, Stanislav, Prof. Dr.**
**Gellertweg 18**
**W-5600 Wuppertal 1(DE)**
Erfinder: **Stasch, Johannes-Peter, Dr.**
**Schneewittchenweg 37**
**W-5600 Wuppertal 1(DE)**
Erfinder: **Knorr, Andreas, Dr.**
**Trillser Graben 10**
**W-4006 Erkrath 2(DE)**
Erfinder: **Wohlfeil, Stefan, Dr.**
**Tucherweg 25**
**W-4010 Hilden(DE)**

(54) **Substituierte Imidazolylpropensäurederivate als Angiotensin II Inhibitoren.**

(57) Die substituierten Imidazolyl-propensäurederivate der allgemeinen Formel (I)

EP 0 539 713 A1

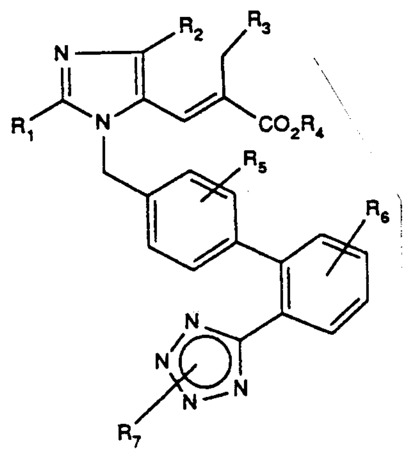

können durch Umsetzung der entsprechenden Aldehyde mit CH-aciden Verbindungen und anschließende Wassereliminierung hergestellt werden. Die substituierten Imidazolyl-propensäurederivate können als Wirkstoffe in Arzneimitteln, insbesondere in blutdrucksenkenden und anti-atherosklerotischen Arzneimitteln eingesetzt werden.

Die Erfindung betrifft neue substituierte Imidazolyl-propensäurederivate, ein Verfahren zu ihrer Herstellung und ihre Verwendung in Arzneimitteln, insbesondere als blutdrucksenkendes und anti-atherosklerotisches Mittel.

Es ist bekannt, daß Renin, ein proteolytisches Enzym, in vivo vom Angiotensinogen das Dekapeptid Angiotensin I abspaltet welches wiederum in der Lunge, den Nieren oder anderen Geweben zu dcm blutdruckerhöhenden Oktapeptid Angiotensin II abgebaut wird. Die verschiedenen Effekte des Angiotensin II, wie beispielsweise Vasokonstriktion, $Na^+$-Retention in der Niere, Aldosteronfreisetzung in der Nebenniere und Tonuserhöhung des sympathischen Nervensystems wirken synergistisch im Sinne einer Blutdruckerhöhung.

Darüber hinaus besitzt Angiotensin II die Eigenschaft, das Wachstum und die Vermehrung von Zellen wie beispielsweise von Herzmuskelzellen und glatten Muskelzellen zu fördern, wobei diese bei verschiedenen Krankheitszuständen (z.B. Hypertonie, Atherosklerose und Herzinsuffizienz) vermehrt wachsen und proliferieren.

Ein möglicher Ansatz zum Eingriff in das Renin-Angiotensin-System RAS) ist neben der Inhibierung der Reninaktivität die Hemmung der Aktivität des Angiotensin-Konversionsenzyms (ACE) sowie die Blockade von Angiotensin II-Rezeptoren.

In den Publikationen EP 324 377 A2, EP 403 158 A2 und EP 403 159 A2 werden Phenyl(alkyl)-imidazole- und Imidazolylalken-Säuren beschrieben, bei denen der Bedeutungsumfang der Position 2 (siehe erfindungsgemäße Verbindungen $-CH=CH-CO_2R^4$) Propensäuren und -ester erfaßt und der endständige Phenylring durch einen Tetrazolring substituiert ist, ohne jedoch einen Hinweis auf einen konkreten Stoffvertreter dieses Substitutionsmusters zu geben.

Die vorliegende Erfindung betrifft jetzt substituierte Imidazolyl-propensäurederivate der allgemeinen Formel (I)

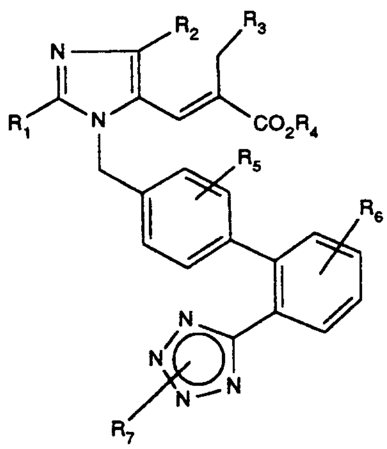

(I)

in welcher

R¹ für geradkettiges oder verzweigtes Alkyl oder Alkenyl mit jeweils bis zu 8 Kohlenstoffatomen steht, die gegebenenfalls durch Cycloalkyl mit 3 bis 6 Kohlenstoffatomen substituiert sind,
für Cycloalkyl mit 3 bis 8 Kohlenstoffatomen steht,

R² für Wasserstoff, Halogen, Hydroxy, Nitro, Cyano, Trifluormethyl, Trifluormethoxy oder für Pentafluorethyl steht, oder
für geradkettiges oder verzweigtes Alkyl mit bis zu 6 Kohlenstoffatomen steht, oder
für Aryl mit 6 bis 10 Kohlenstoffatomen steht,

R³ für Wasserstoff oder für geradkettiges oder verzweigtes Alkyl mit bis zu 10 Kohlenstoffatomen steht, oder
für Aryl mit 6 bis 10 Kohlenstoffatomen oder für einen 5-bis 7-gliedrigen, gesättigten oder ungesättigten Heterocyclus mit bis zu 4 Heteroatomen aus der Reihe S, N oder O steht, die gegebenenfalls bis zu 3-fach gleich oder verschieden durch Halogen, Hydroxy, Nitro, Cyano, Trifluormethyl, Trifluormethoxy oder durch geradkettiges der verzweigtes Alkyl oder Alkoxy mit jeweils bis zu 8 Kohlenstoffatomen oder durch eine Gruppe der Formel $-NR^8R^9$ substituiert sind,
worin

$R^8$ und $R^9$ gleich oder verschieden sind und Wasserstoff, geradkettiges oder verzweigtes Alkyl mit bis zu 6 Kohlenstoffatomen oder Phenyl bedeuten,

$R^4$ für Wasserstoff, geradkettiges oder verzweigtes Alkyl mit bis zu 8 Kohlenstoffatomen oder Phenyl steht,

$R^5$ und $R^6$ gleich oder verschieden sind und für Wasserstoff, Halogen, Cyano, Nitro, Trifluormethyl, Hydroxy, Trifluormethoxy oder für geradkettiges oder verzweigtes Alkyl oder Alkoxy mit jeweils bis zu 6 Kohlenstoffatomen stehen,

$R^7$ für Wasserstoff oder für geradkettiges oder verzweigtes Alkyl mit bis zu 6 Kohlenstoffatomen steht

und deren Salze.

Die erfindungsgemäßen substituierten Imidazlyl-propensäurederivate können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der substituierten Imidazolyl-propensäurederivate können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure` Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen, sein. Besonders bevorzugt sind z.B. Natrium-, Kalium-, Magnesium-oder Calciumsalze, sowie Ammoniumsalze, die abgeleitet sind von Ammoniak oder organischen Aminen wie beispielsweise Ethylamin, Di- bzw. Triethylamin, Di-bzw. Triethanolamin, Dicyclohexylamin, Dimethylaminoethanol, Arginin, Lysin oder Ethylendiamin.

Die erfindungsgemäßen Verbindungen können in stereoisomeren Formen, die sich entweder wie Bild und Spiegelbild (Enantiomere), oder die sich nicht wie Bild und Spiegelbild (Diastereomer) verhalten, existieren. Die Erfindung betrifft sowohl die Enantiomeren oder Diastereomeren oder deren jeweiligen Mischungen. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Heterocyclus steht im allgemeinen für einen 5- bis 7-gliedrigen, bevorzugt 5-bis 6-gliedrigen, gesättigten oder ungesättigten Ring, der als Heteroatome bis zu 2 Sauerstoff-, Schwefel- und/oder Stickstoffatome enthalten kann. Bevorzugt sind 5-und 6-gliedrige Ringe mit einem Sauerstoff-, Schwefel und/oder bis zu 2 Stickstoffatomen. Bevorzugt werden genannt: Thienyl, Furyl, Pyrrolyl, Pyrazolyl, Pyridyl, Pyrimidyl, Pyrazinyl, Pyridazinyl, Thiazolyl, Oxazolyl, Imidazolyl, Pyrrolidinyl, Piperidinyl, Piperazinyl oder Tetrazolyl.

Bevorzugt sind Verbindungen der allgemeinen Formel (I),
in welcher

$R^1$ für geradkettiges oder verzweigtes Alkyl oder Alkenyl mit jeweils bis zu 6 Kohlenstoffatomen steht, die gegebenenfalls durch Cyclopropyl, Cyclobutyl, Cyclopentyl oder Cyclohexyl substituiert sind, für Cyclopropyl, Cyclopentyl oder Cyclohexyl steht,

$R^2$ für Wasserstoff, Fluor, Chlor, Brom, Iod, Trifluormethyl, Trifluormethoxy, Pentafluorethyl, Phenyl oder für geradkettiges oder verzweigtes Alkyl mit bis zu 6 Kohlenstoffatomen steht,

$R^3$ für Wasserstoff oder für geradkettiges oder verzweigtes Alkyl mit bis zu 8 Kohlenstoffatomen steht, oder für Phenyl, Furyl, Thienyl, Imidazolyl, Pyrryl oder Pyridyl steht, die gegebenenfalls bis zu 2-fach gleich oder verschieden durch Fluor, Chlor, Brom, Hydroxy, Nitro, Cyano, Trifluormethyl, Trifluormethoxy oder durch geradkettiges oder verzweigtes Alkyl oder Alkoxy mit jeweils bis zu 6 Kohlenstoffatomen oder durch eien Gruppe der Formel $-NR^8R^9$ substituiert sind,
worin

$R^8$ und $R^9$ gleich oder verschieden sind und Wasserstoff oder geradkettiges oder verzweigtes Alkyl mit bis zu 4 Kohlenstoffatomen bedeuten,

$R^4$ für Wasserstoff oder für geradkettiges oder verzweigtes Alkyl mit bis zu 6 Kohlenstoffatomen steht,

$R^5$ und $R^6$ gleich oder verschieden sind und für Wasserstoff, Fluor, Chlor, Brom, Trifluormethyl, Trifluormethoxy oder für geradkettiges oder verzweigtes Alkyl mit bis zu 4 Kohlenstoffatomen stehen,

$R^7$ für Wasserstoff oder für geradkettiges oder verzweigtes Alkyl mit bis zu 4 Kohlenstoffatomen steht

und deren Salze.

Besonders bevorzugt sind Verbindungen der allgemeinen Formel (I),
in welcher

$R^1$ für geradkettiges oder verzweigtes Alkyl oder Alkenyl mit jeweils bis zu 4 Kohlenstoffatomen oder Cyclopropyl steht,

$R^2$ für Wasserstoff, Fluor, Chlor, Brom, Iod, Trifluormethyl, Pentafluorethyl, Phenyl oder für geradkettiges oder verzweigtes Alkyl mit bis zu 4 Kohlenstoffatomen steht,

$R^3$ für Wasserstoff oder geradkettiges oder verzweigtes Alkyl mit bis zu 6 Kohlenstoffatomen steht, oder für Phenyl, Furyl oder Thienyl steht, die gegebenenfalls durch Fluor, Chlor, Cyano, Hydroxy, Trifluormethyl, Trifluormethoxy oder durch geradkettiges oder verzweigtes Alkyl oder Alkoxy mit jeweils bis zu 4 Kohlenstoffatomen oder durch eine Gruppe der Formel $-NR^8R^9$ substituiert sind,
worin

$R^8$ und $R^9$ gleich oder verschieden sind und Wasserstoff, Methyl oder Ethyl bedeuten,

$R^4$ für Wasserstoff oder für geradkettiges oder verzweigtes Alkyl mit bis zu 4 Kohlenstoffatomen steht,

$R^5$ und $R^6$ gleich oder verschieden sind und für Wasserstoff, Fluor, Chlor oder Brom stehen,

$R^7$ für Wasserstoff, Methyl, Ethyl, Propyl oder Isopropyl steht

und deren Salze.

Ganz besonders bevorzugt sind Verbindungen der allgemeinen Formel (I),
in welcher

$R^1$ für geradkettiges oder verzweigtes Alkyl oder Alkenyl mit jeweils bis zu 4 Kohlenstoffatomen steht,

$R^2$ für Wasserstoff, Fluor, Chlor, Iod, Trifluormethyl oder Pentafluorethyl steht,

$R^3$ für Wasserstoff oder geradkettiges oder verzweigtes Alkyl mit bis zu 4 Kohlenstoffatomen steht, oder für Phenyl, Furyl oder Thienyl steht, die gegebenenfalls durch Fluor, Chlor, Cyano oder Methoxy substituiert sind,

$R^4$ für Wasserstoff, Methyl oder Ethyl steht
und

$R^5$, $R^6$ und $R^7$ für Wasserstoff stehen

und deren Salze.

Außerdem wurde ein Verfahren zur Herstellung der erfindungsgemäßen Verbindungen der allgemeinen Formel (I) mit deren Salze gefunden, dadurch gekennzeichnet, daß man
Aldehyde der allgemeinen Formel (II)

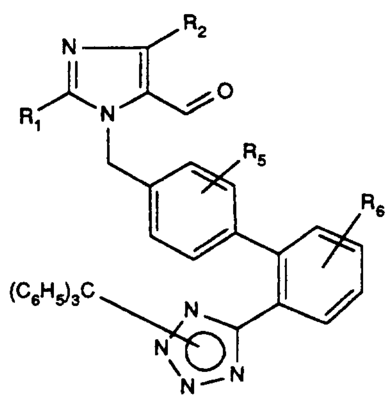

(II)

in welcher
$R^1$, $R^2$, $R^5$ und $R^6$ die oben angegebene Bedeutung haben,
zunächst durch Umsetzung mit Verbindungen der allgemeinen Formel (III)

$R^3-(CH_2)_2-CO_2R^{10}$ (III)

in welcher
$R^3$ die oben angegebene Bedeutung hat
und
$R^{10}$ die oben angegebene Bedeutung von $R^4$ hat aber nicht für Wasserstoff steht,
in inerten Lösemitteln, in Anwesenheit einer Base in die Verbindungen der allgemeinen Formel (IV)

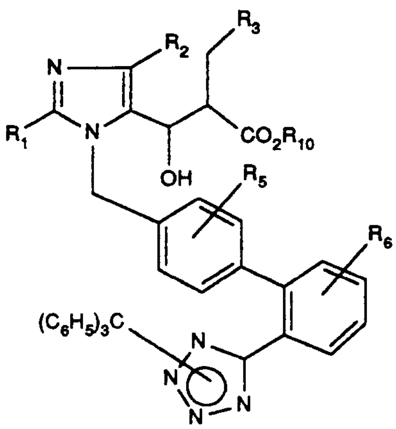

(IV)

in welcher
$R^1$, $R^2$, $R^3$, $R^5$, $R^6$ und $R^{10}$ die oben angegebene Bedeutung haben,
überführt
anschließend die freie Hydroxyfunktion durch Einführung einer Schutzgruppe blockiert und in einem letzten Schritt eine Eliminierung in inerten Lösemitteln in Anwesenheit einer Base durchführt,
und im Fall der Säuren ($R^4$ = H) die Ester verseift,
und im Fall daß $R^7$ nicht für Wasserstoff steht, die $-NH-$Funktion alkyliert.

Das erfindungsgemäße Verfahren kann durch folgendes Formelschema beispielhaft erläutert werden:

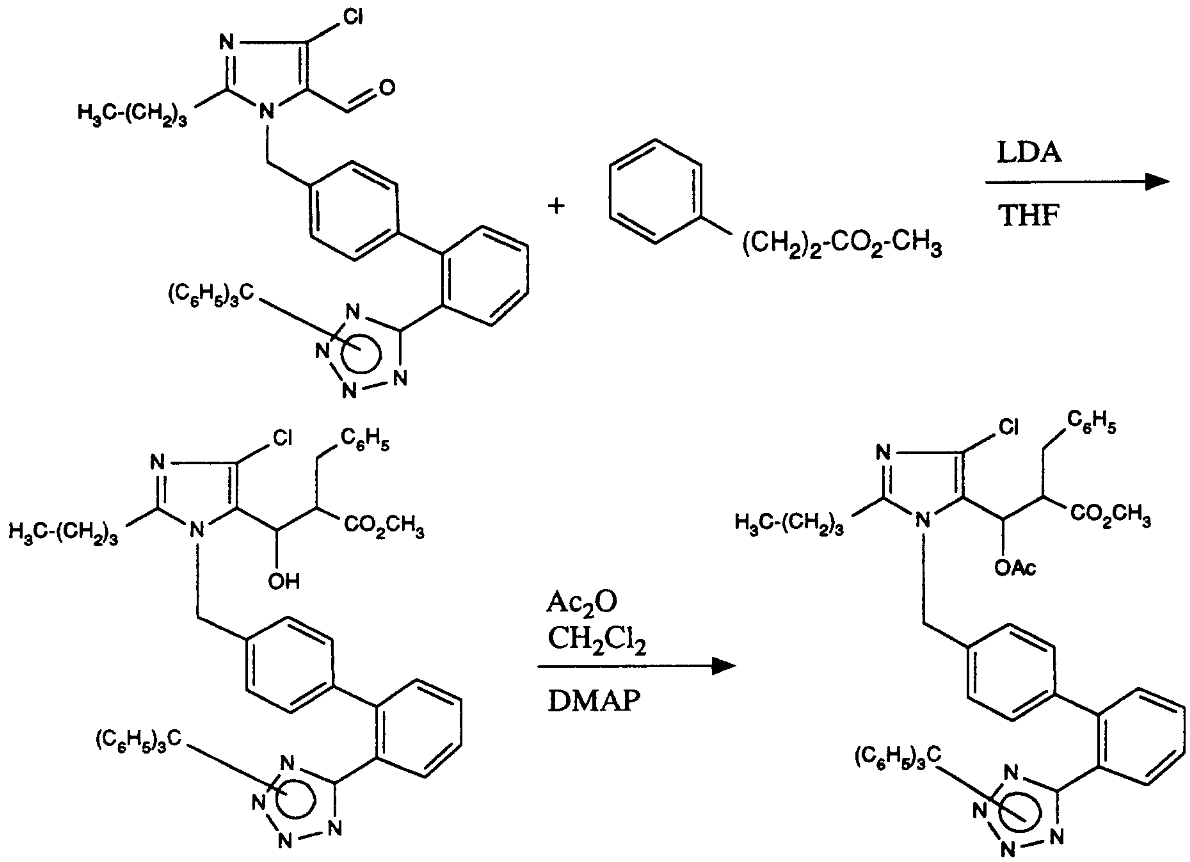
LDA
THF
Ac2O
CH2Cl2
DMAP

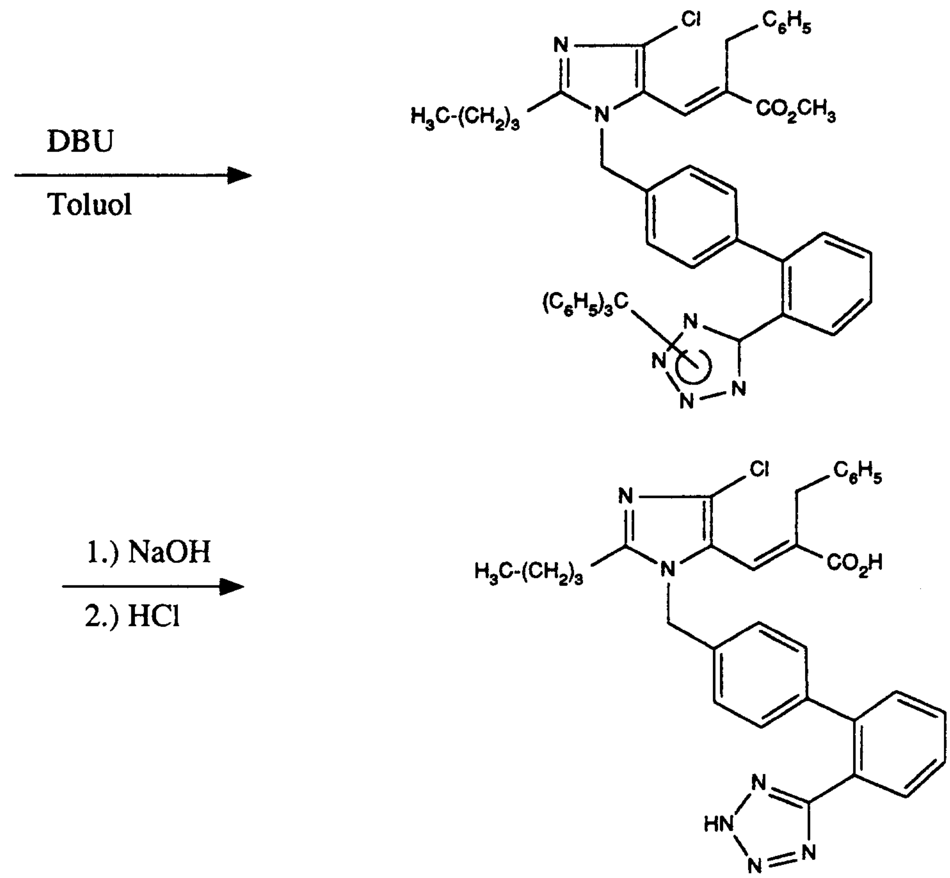


Hydroxyschutzgruppe im Rahmen der oben angegebenen Definition steht im allgemeinen für eine Schutzgruppe aus der Reihe: Benzyloxycarbonyl, Methansulfonyl, Toluolsulfonyl, 2-Nitrobenzyl, 4-Nitrobenzyl, 2-Nitrobenzyloxycarbonyl, 4-Nitrobenzyloxycarbonyl, tert.Butoxycarbonyl, Allyloxycarbonyl, 4-Methoxycarbonyl, Acetyl, Trichloracetyl, 2,2,2-Trichlorethoxycarbonyl, 2,4-Dimethoxybenzyloxycarbonyl, 2-(Methylthiomethoxy)ethoxycarbonyl, Benzoyl, 4-Methylbenzoyl, 4-Nitrobenzoyl, 4-Fluorbenzoyl, 4-Chlorbenzoyl oder 4-Methoxybenzoyl. Bevorzugt sind Acetyl, Methansulfonyl und Toluolsulfonyl.

Als Lösemittel für das Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind für die verschiedenen Schritte Tetrahydrofuran, Methylenchlorid und Toluol.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkali- oder Erdalkalialkoholate oder -amide wie Natrium- oder Kaliummethanolat, Natrium- oder Kaliumethanolat oder Kalium-tert.butylat oder Lithiumdiisopropylamid (LDA), oder organische Amine (Trialkyl($C_1$-$C_6$)amine) wie Triethylamin, oder Heterocyclen wie 1,4-Diazabicyclo-[2.2.2]octan (DABCO), 1,8-Diazabicyclo[5.4.0]undec-7-en (DBU), Pyridin, Diaminopyridin, Methylpiperidin oder Morpholin. Es ist auch möglich, als Basen Alkalimetalle, wie Natrium oder deren Hydride wie Natriumhydrid, einzusetzen. Bevorzugt sind Lithiumdiisopropylamid (LDA) und DBU.

Im allgemeinen setzt man die Base in einer Menge von 0,05 mol bis 10 mol, bevorzugt von 1 mol bis 2 mol bezogen auf 1 mol der Verbindung der Formel (III) ein.

Das erfindungsgemäße Verfahren wird im allgemeinen in einem Temperaturbereich von -100°C bis +100°C, bevorzugt bei -78°C durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Einführung der Schutzgruppe erfolgt im allgemeinen in einem der oben aufgeführten Lösemitteln und einer Base,vorzugsweise in Methylenchlorid mit Dimethylaminopyridin.

Die Blockierung erfolgt im allgemeinen in einem Temperturbereich von 0°C bis +60°C, vorzugsweise bei Raumtemperatur und bei Normaldruck.

Die Eliminierung wird im allgemeinen in einem der oben aufgeführten Lösemitteln, vorzugsweise in Toluol und in Anwesenheit einer der aufgeführten Basen, vorzugsweise DBU durchgeführt.

Die Eliminierung erfolgt im allgemeinen in einem Temperaturbereich von +30°C bis +130°C, vorzugsweise bei +50°C bis +100°C und Normaldruck.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid, oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung kann auch mit Säuren wie beispielsweise Trifluoressigsäure, Essigsäure, Chlorwasserstoffsäure, Bromwasserstoffsäure, Methansulfonsäure, Schwefelsäure oder Perchlorsäure, bevorzugt mit Trifluoressigsäure erfolgen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifüng wird die Base im allgemeinen in einer Menge von 1 bis 3 mol, bevorzugt von 1 bis 1,5 mol bezogen auf 1 mol des Esters, eingesetzt. Besonders bevorzugt verwendet man molare Mengen der Reaktanden.

Bei der Durchführung der Reaktion entstehen im ersten Schritt die Carboxylate der erfindungsgemäßen Verbindungen als Zwischenprodukte, die isoliert werden können. Die erfindungsgemäßen Säuren erhält man durch Behandeln der Carboxylate mit üblichen anorganischen Säuren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren hierbei als vorteilhaft erwiesen, die basische Reaktionsmischung der Verseifung in einem zweiten Schritt ohne Isolierung der Carboxylate anzusäuern. Die Säuren können dann in üblicher Weise isoliert werden. Im Fall der basischen Heterocyclen können durch das Behandeln der Lösungen der Carboxylate mit den oben aufgeführten Säuren auch die Salze der Heterocyclen mit den anorganischen Säuren gewonnen werden.

Die Alkylierung erfolgt im allgemeinen mit Alkylierungsmitteln wie beispielsweise ($C_1-C_6$)-Alkylhalogeniden, Sulfonsäureestern oder substituierten oder unsubstituierten ($C_1-C_6$)-Dialkyl- oder ($C_1-C_6$)-Diarylsulfonate, vorzugsweise Methyljodid oder Dimethylsulfat.

Die Alkylierung erfolgt im allgemeinen in einem der oben aufgeführten Lösemitteln, vorzugsweise in Dimethylformamid in einem Temperaturbereich von 0°C bis +70°C, vorzugsweise von 0°C bis +30°C und Normaldruck.

Die Verbindungen der allgemeinen Formel (II) sind an sich bekannt oder können nach üblicher Methode hergestellt werden [vgl. PCT WO91/00277].

Die Verbindungen der allgemeinen Formel (III) sind bekannt oder können nach üblicher Methode hergestellt werden [vgl. z.B. Beilstein 9, 511].

Die Verbindungen der allgemeinen Formel (IV) sind als konkrete Stoffvertreter neu und können beispielsweise nach dem oben aufgeführten Verfahren hergestellt werden.

Die vorstehenden Herstellungsverfahren sind lediglich zur Verdeutlichung angegeben. Die Herstellung der erfindungsgemäßen Verbindungen der allgemeinen Formel (I) ist nicht auf diese Verfahren beschränkt, jede Modifikation dieser Verfahren ist in gleicher Weise für die Herstellung anwendbar.

Die erfindungsgemäßen substituierten Imidazolyl-propensäurederivate zeigen ein nicht vorhersehbares, wertvolles pharmakologisches Wirkspektrum.

Die erfindungsgemäßen Verbindungen besitzen eine spezifische A II-antagonistische Wirkung, da sie kompetitiv die Bindung von Angiotensin II an die Rezeptoren hemmen. Sie unterdrücken die vasokonstriktorischen und Aldosteronsekretionsstimulierenden Effekte des Angiotensin II. Darüberhinaus inhibieren sie die Proliferation von glatten Muskelzellen.

Sie können deshalb in Arzneimittel zur Behandlung der arteriellen Hypertonie und Atherosklerose eingesetzt werden. Darüberhinaus können sie zur Behandlung von coronaren Herzerkrankungen, Herzinsuffizienz, Störungen der Hirnleistung, ischämischen Gehirnerkrankungen, peripherer Durchblutungsstörungen, Funktionsstörungen der Niere und Nebenniere, bronchospastischen und vaskulär bedingten Erkrankungen der Atemwege, Natriumretention und Ödemen eingesetzt werden.

Untersuchung auf die Hemmung der mit Agonisten induzierten Kontraktion

Kaninchen beiderlei Geschlechts werden durch Nackenschlag betäubt und entblutet, oder fallweise mit Nembutal (ca. 60 - 80 mg/kg i.v.) narkotisiert und durch Öffnung des Thorax getötet. Die Thoraxaorta wird entnommen, von anhaftendem Bindegewebe befreit, in 1,5 mm breite Ringsegmente geteilt und einzeln unter einer Anfangsbelastung von ca. 3,5 g in 10 ml Organbäder mit auf 37°C temperierter, Carbogen-begaster Krebs-Henseleit-Nährlösung folgender Zusammensetzung: 119 mmol/l NaCl; 2,5 mmol/l $CaCl_2$ x 2 $H_2O$; 1,2 mmol/l $KH_2PO_4$; 10 mmol/l Glucose; 4,8 mmol/l KCl; 1,4 mmol/l $MgSO_4$ x 7 $H_2O$ und 25 mmol/l $NaHCO_3$ verbracht.

Die Kontraktionen werden isometrisch durch Statham UC2-Zellen über Brückenverstärker (ifd Mülheim bzw. DSM Aalen) erfaßt und mittels A/D-Wandler (System 570, Keithley München) digitalisiert sowie ausgewertet. Die Durchführung von Agonistdosiswirkungskurven (DWK) erfolgt stündlich. Mit jeder DWK werden 3 bzw. 4 Einzelkonzentrationen im 4 min-Abstand in die Bäder appliziert. Nach Ende der DWK und darauffolgender Auswaschzyklen (16 mal jeweils ca. 5 sec/min mit der o.a. Nährlösung) schließt sich eine 28-minütige Ruhe- bzw. Inkubationsphase an, innerhalb derer die Kontraktionen in der Regel wieder den Ausgangswert erreichen.

Die Höhe der im Normalfall 3. DWK wird als Bezugsgröße für die Bewertung der in weiteren Durchgängen zu untersuchenden Testsubstanz verwendet, die bei den nachfolgenden DWK's in jeweils steigender Dosierung mit Beginn der Inkubationszeit in die Bäder appliziert wird. Jeder Aortenring wird dabei ganztägig mit immer dem gleichen Agonisten stimuliert.

Agonisten und ihre Standardkonzentrationen (Applikationsvolumen pro Einzelgabe = 100 µl):

| | | |
|---|---|---|
| KCl | 22,7;32,7;42,7;52,7 | mmol/l |
| 1Noradrenalin | $3x10^{-9}$;$3x10^{-8}$;$3x10^{-7}$;$3x10^{-6}$ | g/ml |
| Serotonin | $10^{-8}$;$10^{-7}$;$10^{-6}$;$10^{-5}$ | g/ml |
| B-HT 920 | $10^{-7}$;$10^{-6}$;$10^{-5}$ | g/ml |
| Methoxamin | $10^{-7}$;$10^{-6}$;$10^{-5}$ | g/ml |
| Angiotensin II | $3x10^{-9}$;$10^{-8}$;$3x10^{-8}$;$10^{-7}$ | g/ml |

Für die Berechnung der $IC_{50}$ (Konzentration bei der die zu untersuchende Substanz eine 50%ige Hemmung verursacht) wird der Effekt jeweils an der 3. = submaximalen Agonistkonzentration zugrundegelegt.

Die erfindungsgemäßen Verbindungen hemmen die durch Angiotensin II induzierte Kontraktion der isolierten Kaninchenaorta dosenabhängig. Die durch Kalium-Depolarisation oder andere Agonisten induzierte Kontraktion wurde nicht oder in hohen Konzentrationen nur schwach gehemmt.

Blutdruckmessungen an der Angiotensin II-infundierten Ratte

Männliche Wistar Ratten (Moellegaard, Kopenhagen, Dänemark) mit einem Körpergewicht von 300 - 350 g, werden mit Thiopental (100 mg/kg i.p.) anästhesiert. Nach Tracheotomie wird in die Femoralarterie ein Katheter zur Blutdruckmessung und in die Femoralvenen ein Katheter für die Angiotensin II-Infusion und ein Katheter für die Substanzverabreichung eingeführt. Nach Gabe des Ganglienblockers Pentolinium (5 mg/kg i.v.) wird die Angiotensin II-Infusion (0,3 µg/kg/min) gestartet. Sobald die Blutdruckwerte ein stabiles Plateau erreicht haben, werden die Testsubstanzen entweder intravenös oder als Suspension bzw. Lösung in 0,5% Tylose oral verabreicht. Die Blutdruckveränderungen unter Substanzeinfluß sind als Mittelwerte ±

SEM in der Tabelle angegeben.

Bestimmung der antihypertensiven Wirksamkeit bei wachen hypertensiven Ratten

Die orale antihypertensive Wirksamkeit der erfindungsgemäßen Verbindungen wurde an wachen Ratten mit chirurgisch induzierter unilateraler Nierenarterienstenose geprüft. Dazu wurde die rechte Nierenarterie mit einem Silberclip von 0,18 mm lichter Weite eingeengt. Bei dieser Hypertonieform ist die Plasmareninaktivität in den ersten sechs Wochen nach dem Eingriff erhöht Der arterielle Blutdruck diese Tiere wurde in definierten Zeitabständen nach Substanzgabe mit der "Schwanzmanschette" unblutig gemessen. Die zu prüfenden Substanzen wurden aufgeschwemmt in einer Tylosesuspension intragastral ("oral") per Schlundsonde in verschiedenen Dosen appliziert. Die erfindungsgemäßen Verbindungen senken den arteriellen Blutdruck der Hochdruckratten in klinisch relevanter Dosierung.

Außerdem hemmen die erfindungsgemäßen Verbindungen die spezifische Bindung von radioaktivem Angiotensin II konzentrationsabhängig.

Interaktion der erfindungsgemäßen Verbindungen mit dem Angiotensin II-Rezeptor an Membranfraktionen der Nebennierenrinde Rind)

Nebennierenrinden vom Rind (NNR), die frisch entnommen und sorgfältig von Mark von Kapsel befreit sind, werden in Sucrose-Lösung (0,32 M) mit Hilfe eines Ultra-Turrax (Janke & Kunkel, Staufen i.B.) zu grobem Membran-Homogenat zerkleinert und in zwei Zentrifugationsschritten zu Membranfraktionen partiell aufgereinigt.

Die Untersuchungen zur Rezeptor-Bindung werden an partiell gereinigten Membranfraktionen boviner NNR mit radioaktivem Antiotensin II in einem Assay-Volumen von 0,25 ml durchgeführt, das im einzelnen die partiell gereinigten Membranen (50 - 80 µg), $^3H$-Angiotensin II(3-5 nM), Test-Pufferlösung (50 mM Tris, pH 7,2, 5 mM $MgCl_2$, 0,25% BSA) sowie die zu untersuchenden Substanzen enthält. Nach einer Inkubationszeit von 60 min bei Raumtemperatur wird die nicht gebundene Radioaktivität der Proben mittels angefeuchteter Glasfaserfilter (Whatman GF/C) separiert und die gebundene Radioaktivität nach Waschung des Proteins mit eiskalter Pufferlösung (50 mM Tris/HCl, pH 7,4, 5% PEG 6000) in einem Szintillationscocktail spektrophotometrisch gemessen. Die Analyse der Rohdaten erfolgte mit Computer-Programmen zu $K_i$- bzw. $IC_{50}$-Werten ($K_i$: für die verwendete Radioaktivität korrigierte $IC_{50}$-Werte; $IC_{50}$-Werte: Konzentration bei der die zu untersuchende Substanz eine 50%ige Hemmung der totalen Bindung des Radioliganden bewirkt).

Untersuchung zur Inhibition der Proliferation glatter Muskelzellen durch die erfindungsgemäßen Verbindungen

Zur Feststellung der antiproliferativen Wirkung der Verbindungen werden glatte Muskelzellen verwendet, die aus den Aorten von Ratten durch die Media-Explantat-Technik gewonnen werden [R. Ross, J. Cell. Biol. 50, 172, 1971]. Die Zellen werden in geeigneten Kulturschalen, in der Regel 24-Loch-Platten, ausgesät und für 2 - 3 Tage in Medium 199 mit 7,5% FCS und 7,5% NCS, 2 mM L-Glutamin und 15 mM HEPES, pH 7,4 in 5% $CO_2$ bei 37°C kultiviert. Danach werden die Zellen durch Serumentzug für 2 - 3 Tage synchronisiert und sodann mit AII, Serum oder anderen Faktoren zum Wachstum angeregt. Gleichzeitig werden Testverbindungen zugesetzt. Nach 16 - 20 Stunden wird 1 µCi $^3H$-Thymidin zugefügt und nach weiteren 4 Stunden der Einbau dieser Substanz in die TCA-präzipitierbare DNA der Zellen bestimmt.

Der neue Wirkstoff kann in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermittlen und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannnte obere Grenze überschrittten werden muß. Im Falle der Applikation größerer Mengen kann es empfehlenswert sein, diese in mehreren Einzelgaben über den Tag zu verteilen.

Ausgangsverbindungen

Beispiel I

2-Benzyl-3-[2-n-butyl-4-chlor-1-{(2'- (N-triphenylmethyl-tetrazol-5-yl)biphenyl-4-yl)-methyl} - 1H-imidazol-5-yl]-3-hydroxy-propionsäure-methylester

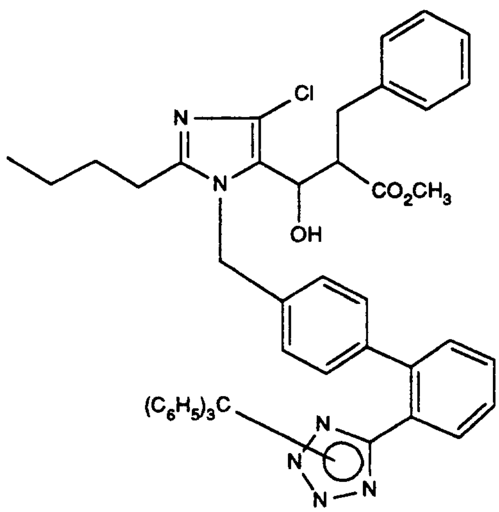

180,4 mg (1,1 mmol) 3-Phenylpropionsäuremethylester werden in 2 ml THF gelöst, anschließend werden bei -78°C 0,8 ml einer 1,5 M Lösung von Lithiumdiisopropylamid in Cyclohexan (1,2 mmol) zugegeben. Es wird 30 min bei -78°C gerührt, 662,5 mg (1,0 mmol) 2-n-Butyl-4-chlor-1-[(2'-(N-triphenylmethyltetrazol-5-yl)biphenyl-4-yl)methyl]-1H-imidazol-5-carboxaldehyd in 5 ml THF zugegeben, 30 min bei -78°C gerührt, bei 0°C 5 ml gesättigte Ammoniumchlorid-Lösung zugegeben und dreimal mit 20 ml Ether extrahiert. Die organische Phase wird über Natriumsulfat getrocknet und der Rückstand über Kieselgel 60 mit Essigeste/Petrolether (1:1) chromatographiert.

Ausbeute: 300 mg (36% der Theorie)

$R_f$ = 0,53 und 0,48 Diastereomerengemisch (Essigester : Petrolether 1:1)

Beispiel II

(E) – 2 – Benzyl – 3 – [2 – n – butyl – 4 – chlor – 1 – {(2' – (N – triphenylmethyl – tetrazol – 5 – yl)biphenyl – 4 – yl)methyl} – 1H – imidazol – 5 – yl] – 2 – propensäure – methylester

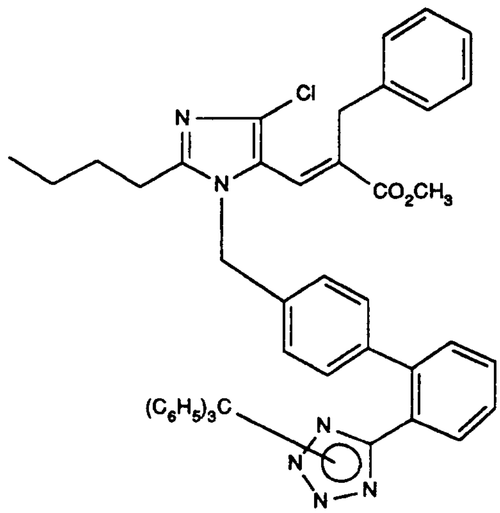

300 mg (0,36 mmol) der Verbindung aus Beispiel I werden in 3 ml Dichlormethan gelöst, anschließend werden nacheinander 16 mg (0,13 mmol) N,N – Dimethylaminopyridin (DMAP) und 41 mg (0,4 mmol) Acetanhydrid zugegeben, 1 h bei 25 °C gerührt, 2 ml Wasser und 10 ml Ether zugegeben. Die organische Phase wird nacheinander mit je 3 ml gesättiger Natriumhydrogencarbonat – Lösung und gesättiger Natriumchlorid – Lösung extrahiert, über Natriumsulfat getrocknet und eingeengt. Das so erhaltene Rohprodukt wird in 3 ml Toluol gelöst, anschließend werden 183 mg (1,2 mmol) 1,8 – Diazabicyclo[5.4.0]undec – 7 – en (DBU) zugegeben und 20 h bei 90 °C gerührt. Nach Abkühlen werden 3 ml Ether zugegeben, mit 5 ml gesättiger Natriumchlorid – Lösung extrahiert, die organische Phase eingeengt und der Rückstand über Kieselgel 60 mit Essigester / Petrolether (1:2) chromatographiert.
Ausbeute: 120 mg (41% der Theorie)
$R_f$ = 0,63 (Essigester: Petrolether 1:1)

Beispiel III

(E) – 3 – [2 – n – Butyl – 4 – chlor – 1 – {(2' – (N – triphenylmethyltetrazol – 5 – yl) – biphenyl – 4 – yl) – methyl } – 1H – imidazol – 5 – yl] – 2 – (2 – methylpropyl) – 2 – propensäureethylester

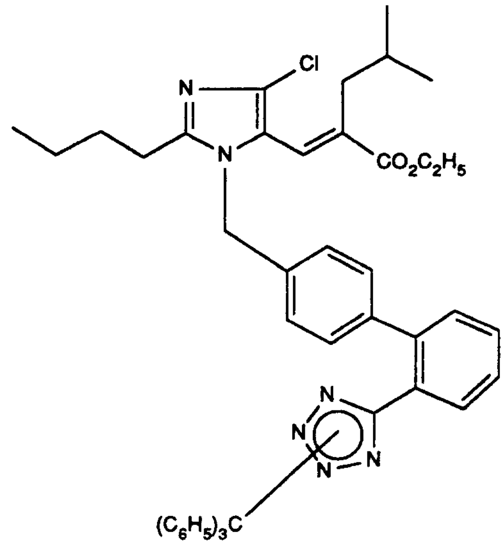

In Analogie zur Vorschrift des Beispiels II wurde die Titelverbindung aus 1,72 g (2,13 mmol) 3 – [2 – n – Butyl – 4 – chlor – 1 – {(2' – (N – triphenylmethyltetrazol – 5 – yl)biphenyl – 4 – yl)methyl} – 1H – imidazol – 5 – yl] – 3 – hydroxy – 2 – (2 – methylpropyl) – 2 – propensäureethylester hergestellt.
Ausbeute: 440 mg (27 % der Theorie)
$R_f$ = 0,71 (Essigester/Petrolether = 1:2)

Beispiel IV

2 – n – Butyl – 1 – [(2' – N – triphenylmethyl – tetrazol – 5 – yl]biphenyl – 4 – yl)methyl] – 1H – imidazol – 5 – carboxaldehyd

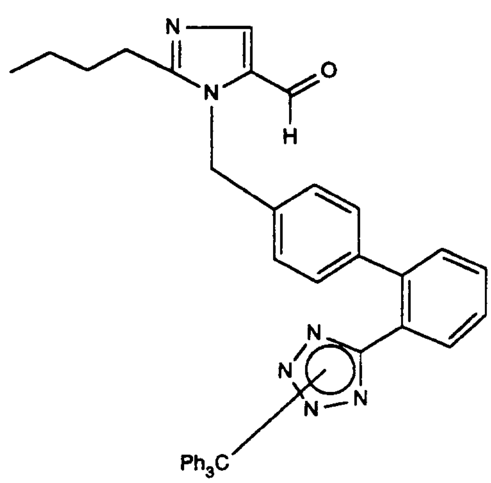

Eine Lösung von 12,0 g (18,1 mmol) 2 – n – Butyl – 4 – chlor – 1[(2' – (N – triphenylmethyltetrazol – 5 – yl]biphenyl – 4 – yl)methyl] – 1H – imidazol – 5 – carboxaldehyd in 150 ml Methanol wird bei 25 °C in Gegenwart von 1,2 g Palladium auf Kohle (5%ig) und 2,46 g (18,1 mmol) Natriumacetat – Trihydrat 1,5 h bei ca. 3 bar Wasserstoffdruck hydriert. Danach wird vom Katalysator abfiltriert, eingeengt und der Rückstand

an Kieselgel mit Essigester/Petrolether (1:1) chromatographiert.
Ausbeute: 3,85 g (34 % der Theorie)
$R_f$ = 0,41 (Essigester/Petrolether = 1:1)

Beispiel V

3 – [2 – n – Butyl – 1 – {(2' – (N – triphenylmethyltetrazol – 5 – yl)biphenyl – 4 – yl)methyl} – 1H – imidazol – 5 – yl] – 2 – (2 – methylpropyl) – 3 – hydroxy – propionsäure – ethylester

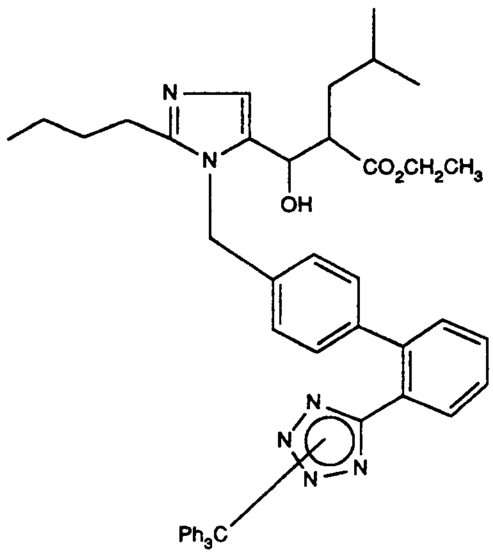

Zu einer Lösung von 0,89 g (8,75 mmol) N,N – Diisopropylamin in 10 ml THF werden unter Schutzgas bei – 78 ° C 5,15 ml (8,25 mmol) einer 1,6 N Lösung von n – Butyllithium in n – Hexan injiziert. Anschließend wird die Reaktionslösung kurz auf 0 ° C erwärmt, erneut auf – 78 ° C gekühlt und 1,24 ml (7,5 mmol) Isocapronsäureethylester in 5 ml THF zugegeben. Es wird 30 min bei – 78 ° C gerührt, 3,14 g (5 mmol) der Verbindung aus Beispiel IV in 20 ml THF zugegeben und 1 h bei – 78 ° C nachgerührt. Danach wird langsam auf 25 ° C erwärmt, 20 mi ges. Ammoniumchlorid – Lösung zugegeben und dreimal mit je 50 ml Essigester extrahiert. Die organische Phase wird über Natriumsulfat getrocknet, eingeengt und der Rück – stand an Kieselgel mit Essigester/Petrolether (3:1) gereinigt.
Ausbeute: 1,82 g (47 % der Theorie)
$R_f$ = 0,27 (Essigester/Petrolether = 2:1, Diastereomerengemisch)

Beispiel VI

3 – Acetoxy – 3 – [2 – n – butyl – 1 – {(2' – (N – triphenylmethyltetrazol – 5 – yl)biphenyl – 4 – yl) – methyl] – 1H – imidazol – 5 – yl] – 2 – (2 – propylmethyl) – propionsäure – ethylester

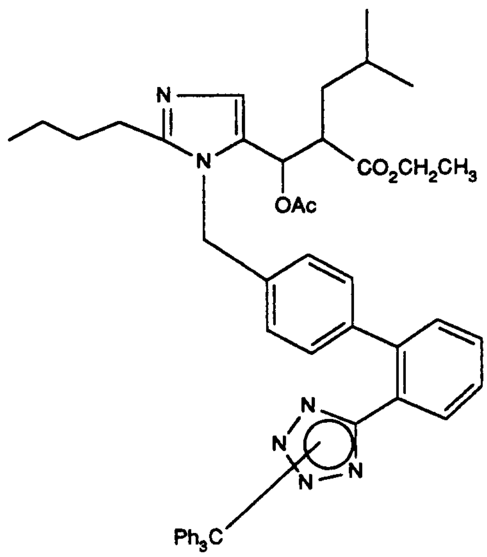

11,1 g (14,4 mmol) der Verbindung aus Beispiel V werden in 100 ml Dichlormethan gelöst, mit 626 mg (5,13 mmol) N,N' – Dimethylaminopyridin (DMAP) und 2,04 ml (21,6 mmol) Acetanhydrid versetzt und 16 h bei 25 ° C gerührt. Man verdünnt mit Ether, wäscht mit Wasser (1 x 50 ml), ges. Natriumhydrogencarbonat – Lösung (1 x 50 ml) und ges. Natriumchlorid – Lösung (1 x 50 ml), trocknet die organische Phase über Natriumsulfat und engt ein. Das so erhaltene Rohprodukt wird an Kieselgel mit Essigester/Petrolether (2:1) chromatographiert.
Ausbeute: 11,7 g (100% der Theorie)
$R_f$ = 0,52 (Essigester/Petrolether = 2:1, Diastereomerengemisch)

Beispiel VII

3 – [2 – n – butyl – 1 – {2' – (N – triphenylmethyltetrazol – 5 – yl)biphenyl – 4 – yl)methyl} – 1H – imidazol – 5 – yl] – 2 – (2 – methylpropyl) – 2 – propensäure – ethylester

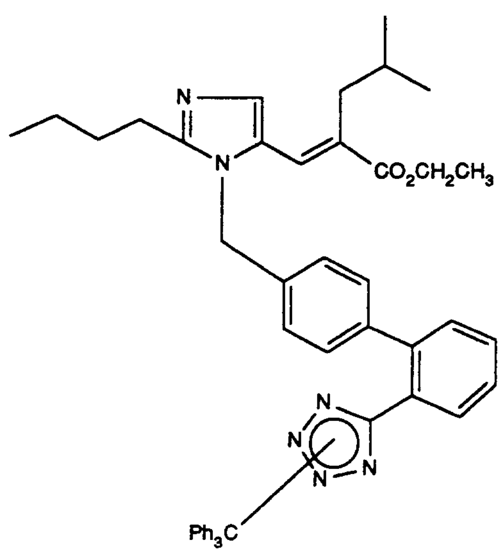

11,7 g (14,3 mmol) der Verbindung aus Beispiel VI werden in 150 ml Toluol gelöst, 5,3 ml (35,8 mmol) 1,8-Diazabicyclo[5.4.0]undec-7-en (DBU) zugegeben und 5 h am Rückfluß gekocht. Danach werden erneut 2 ml (13,5 mmol) DBU zugegeben und weitere 2,5 h am Rückfluß gekocht. Nach Abkühlen wird mit ges. Natriumchlorid-Lösung (1 x 70 ml) gewaschen, die organische Phase über Natriumsulfat getrocknet, filtriert und eingeengt. Der Rückstand wird an Kieselgel mit Essigester/Petrolether (1:1) chromatographiert.
Ausbeute: 3 g (28 % der Theorie)
$R_f$ = 0,68 (Essigester/Petrolether = 1:1)

Herstellungsbeispiele

Beispiel 1

(E)-2-Benzyl-3-[2-n-butyl-4-chlor-1-{(2'-(tetrazol-5-yl)biphenyl-4-yl)methyl}-1H-imidazol-5-yl]-2-propensäure

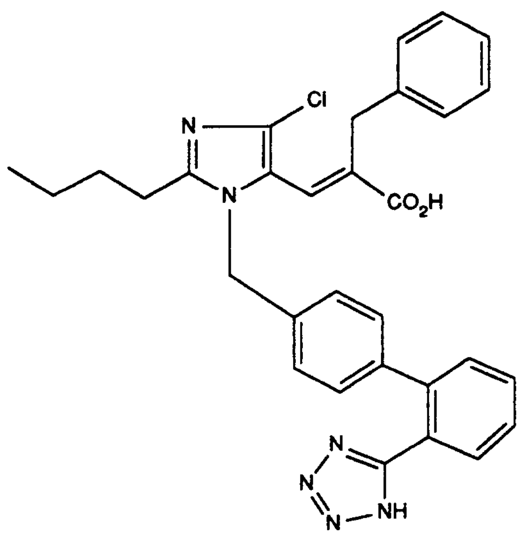

81 mg (0,1 mmol) der Verbindung aus Beispiel II werden in 3 ml 2 N methanolischer Natronlauge gelöst, 30 min zum Sieden erhitzt, nach Abkühlung mit konz. Salzsäure auf pH 1 angesäuert und mit 20 ml Dichlormethan extrahiert. Nach Einengen wird der Rückstand über Kieselgel 60 mit Dichlormethan / Methanol / Eisessig (10:1:0,05) chromatographiert.
Ausbeute: 37 mg (67% der Theorie)
$R_f$ = 0,40 (Dichlormethan / Methanol / Eisessig = 10:1:0,05)

Beispiel 2

(E) – 3 – [2 – n – Butyl – 4 – chlor – 1 – {[2'(tetrazol – 5 – yl)biphenyl – 4 – yl) – methyl} – 1H – imidazol – 5 – yl] – 2 – (4 – methoxybenzyl) – 2 – propensäure

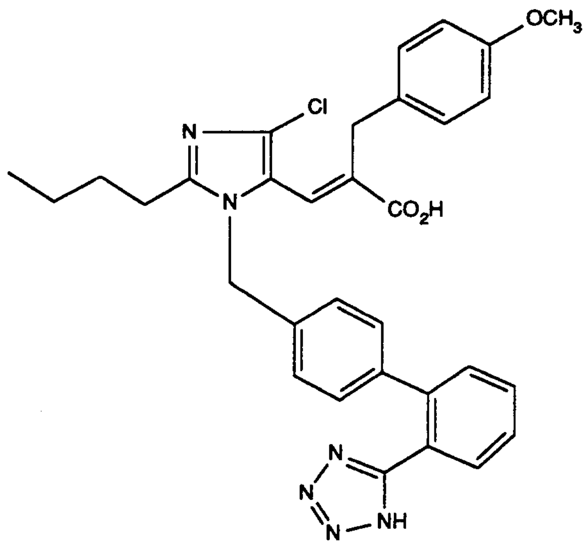

Zu einer Lösung von 356 mg (0,42 mmol) 3 – [2 – n – Butyl – 4 – chlor – 1 – {(2' – (N – triphenylmethyltetrazol – 5 – yl)biphenyl – 4 – yl)methyl} – 1H – imidazol – 5 – yl] – 2 – (4 – methoxybenzyl) – 2 – propensäuremethylester in 6 ml THF werden 2 ml Wasser und 2 ml Trifluoressigsäure gegeben. Es wird 6 h bei 25 ° C gerührt, eingeengt, mit Dioxan/Wasser (1:1) aufgenommen und mit konz. Lithiumhydroxidlö – sung alkalisch gestellt. Die Reaktionsmischung wird 3 h bei 25 ° C gerührt, mit Essigester ausgeschüttelt, die wäßrige Phase mit verd. Salzsäure angesäuert und erneut mit Essigester extrahiert. Die organische Phase wird über Natriumsulfat getrocknet, eingeengt und der Rückstand über Kieselgel 60 mit Dichlormethan/Methanol (8:1) chromatographiert.
Ausbeute: 230 mg (94 % der Theorie)
$R_f$ = 0,63 (Dichlormethan/Methanol = 8:1)

Beispiel 3

(E) – 3 – [2 – n – Butyl – 4 – chlor – 1 – {(2' – (tetrazol – 5 – yl)biphenyl – 4 – yl)methyl} – 1H – imidazol – 5 – yl] – 2 – (2 – methylpropyl) – 2 – propensäureethylester

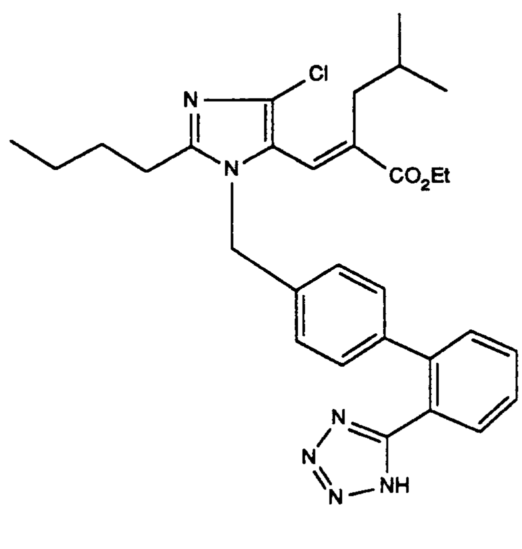

Zu einer Lösung von 440 mg (0,56 mmol) der Verbindung aus Beispiel III in 10 ml THF werden nacheinander 1,5 ml $H_2O$ und 1,5 ml Trifluoressigsäure gegeben. Es wird 24 h bei 25 ° C gerührt, mit konz. Natronlauge basisch gestellt, mit 20 ml Ether gewaschen, die wäßrige Phase mit halbkonz. Salzsäure angesäuert und dreimal mit je 30 ml Essigester extrahiert. Die organischen Phasen werden über Natriumsulfat getrocknet, eingeengt und der Rückstand an Kieselgel 60 mit Dichlormethan/Methanol (10:1) chromatographiert.
Ausbeute: 130 mg (43 % der Theorie)
$R_f$ = 0,31 (Dichlormethan/Methanol = 10:1)

Beispiel 4

(E)-3-[2-n-Butyl-4-chlor-1-{(2'-(tetrazol-5-yl)biphenyl-4-yl)methyl]-1H-imidazol-5-yl]-2-(2-thienylmethyl)-2-propensäure

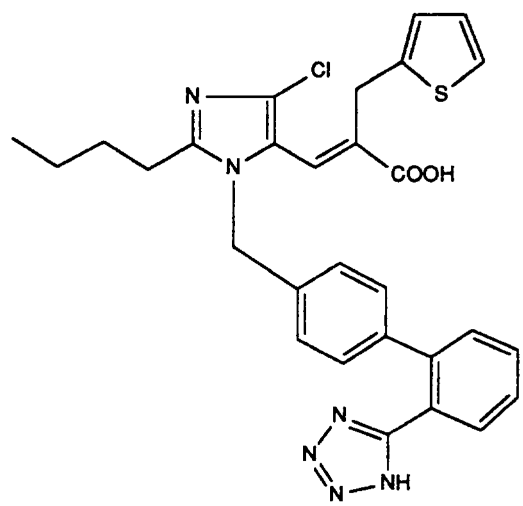

In Analogie zur Vorschrift des Beispiels 1 wurde die Titelverbindung aus 410 mg (0,72 mmol) (E)-3-[2-n-Butyl-4-chlor-1-{(2'-(tetrazol-5-yl)biphenyl-4-yl)methyl}--1H-imidazol-5-yl]-2-(2-thienylmethyl)-2-propensäuremethylester hergestellt.
Ausbeute: 110 mg (28 % der Theorie)
$R_f$ = 0,27 (Dichlormethan/Methanol = 10:1)

Beispiel 5

3 – [2 – n – Butyl – 1 – {2' – (tetrazol – 5 – yl)biphenyl – 4 – yl)methyl} – 1H – imidazol – 5 – yl] – 2 – (2 – methylpropyl) – 2 – propensäure – ethylester

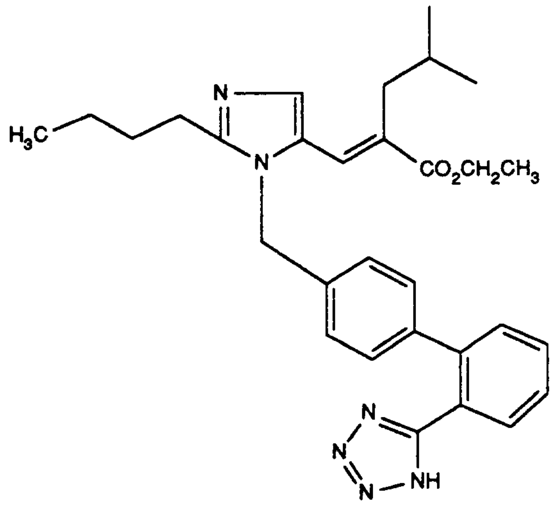

Eine Lösung von 3,0 g (3,9 mmol) der Verbindung aus Beispiel VII in 30 ml Methanol wird langsam mit 2 ml konz. Salzsäure versetzt. Nach 15 min gießt man die Reaktionslösung auf 300 ml Wasser, extrahiert mit Dichlormethan (4 x 70 ml), trocknet die organische Phase über Natriumsulfat, filtriert und engt ein. Der Rückstand wird an Kieselgel mit Toluol/Methanol/Eisessig (35:5:0,5) chromatographiert.
Ausbeute: 1,88 g (94 % der Theorie)
$R_f$ = 0,21 (Toluol/Methanol/Eisessig = 35:5:0,2)

Beispiel 6

3 – [2 – n – Butyl – 1 – {(2'tetrazol – 5 – yl)biphenyl – 4 – yl)methyl} – 1H – imidazol – 5 – yl] – 2 – (2 – methylpropyl) – 2 – propensäure

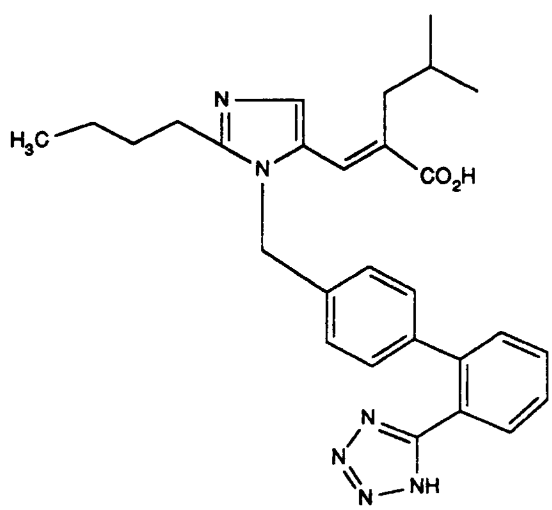

Zu einer Lösung von 1,44 g (2,8 mmol) der Verbindung aus Beispiel 5 in 50 ml Methanol gibt man eine Lösung von 1 g Natriumhydroxid in 10 ml Methanol und rührt 16 h bei 50 ° C. Nach Abkühlen wird mit verd. Salzsäure sauer gestellt, mit Dichlormethan (3 x 75 ml) und Essigester (3 x 75 ml) extrahiert, die vereinigten

organischen Phasen über Natriumsulfat getrocknet, filtriert und eingeengt. Der Rückstand wird an Kieselgel mit Toluol/Methanol/Eisessig (35:5:0,2) chromatographiert.
Ausbeute: 0,41 g (30 % der Theorie)
$R_f$ = 0,12 (Toluol/Methanol/Eisessig = 35:5:0,2)
In Analogie zu den oben aufgeführten Vorschriften werden die in Tabelle 1 gezeigten Verbindungen hergestellt.

Allgemeine Arbeitsvorschrift zur Herstellung der Salze:

Eine Lösung der betreffenden Imidazolyl-propensäure in Dioxan/Wasser wird mit equimolaren Mengen einer 1 N NaOH neutralisiert, eingefroren und über Nacht lyophilisiert.

Tabelle 1:

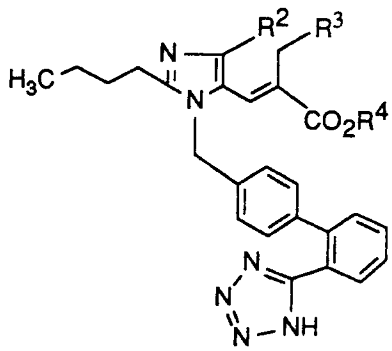

| Beisp.Nr. | $R^2$ | $R^3$ | $R^4$ | Isomer | $R_f$ |
|---|---|---|---|---|---|
| 7 | Cl | 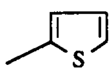 | H | (Z) | 0,32[b)] |
| 8 | H | 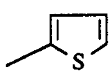 | $-CH_3$ | | 0,18[c)] |
| 9 | H | 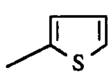 | H | | 0,17[d)] |
| 10 | H | 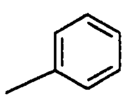 | H | | 0,19[a)] |
| 11 | H | 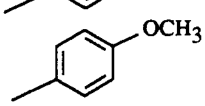 | H | | 0,15[d)] |
| 12 | H | 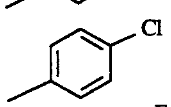 | H | | 0,16[d)] |
| 13 | H | 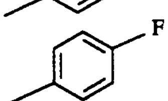 | H | | 0,16[d)] |
| 14 | H | 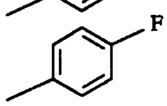 | $-CH_3$ | | 0,24[d)] |

a) Dichlormethan/Methanol = 10:1
b) Dichlormethan/Methanol = 5:1
c) Toluol/Methanol/Eisessig = 35:5:0,2
d) Toluol/Methanol/Eisessig = 35:5:1
e) Dichlormethan/Methanol/Eisessig = 10:1:0,05

Die Verbindungen in Tabelle 2 werden nach der oben angegebenen Arbeitsvorschrift hergestellt.

Tabelle 2:

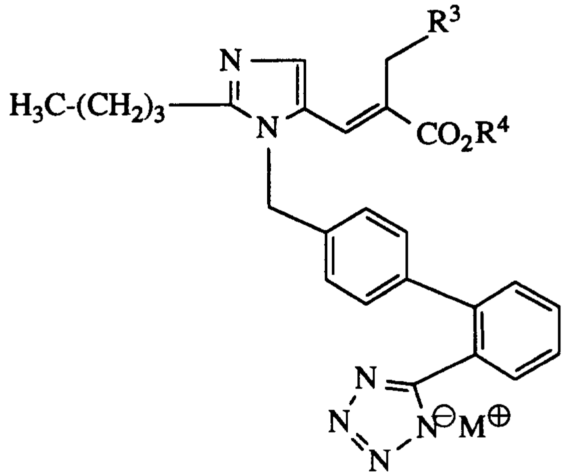

| Beisp.Nr. | $R^3$ | $R^4$ | M |
|---|---|---|---|
| 15 | $CH(CH_3)_2$ | $C_2H_5$ | Na |
| 16 | $CH(CH_3)_2$ | Na | Na |
| 17 | 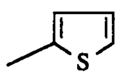 | $CH_3$ | Na |
| 18 | 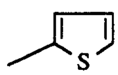 | Na | Na |
| 19 | 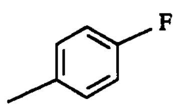 | $CH_3$ | Na |
| 20 | 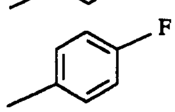 | Na | Na |

## Patentansprüche

1. Substituierte Imidazolyl – propensäurederivate der allgemeinen Formel

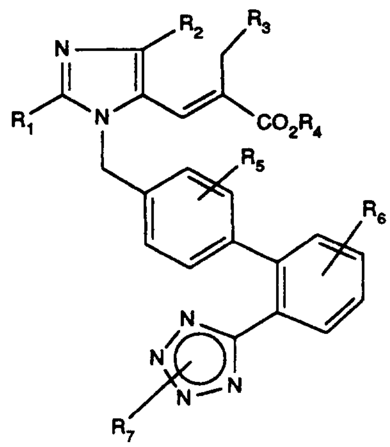

(I)

in welcher

$R^1$ für geradkettiges oder verzweigtes Alkyl oder Alkenyl mit jeweils bis zu 8 Kohlenstoffatomen steht, die gegebenenfalls durch Cycloalkyl mit 3 bis 6 Kohlenstoffatomen substituiert sind,
für Cycloalkyl mit 3 bis 8 Kohlenstoffatomen steht,

$R^2$ für Wasserstoff, Halogen, Hydroxy, Nitro, Cyano, Trifluormethyl, Trifluormethoxy oder für Pentafluorethyl steht, oder
für geradkettiges oder verzweigtes Alkyl mit bis zu 6 Kohlenstoff-atomen steht, oder
für Aryl mit 6 bis 10 Kohlenstoffatomen steht,

$R^3$ für Wasserstoff oder für geradkettiges oder verzweigtes Alkyl mit bis zu 10 Kohlenstoffatomen steht, oder
für Aryl mit 6 bis 10 Kohlenstoffatomen oder für einen 5-bis 7-gliedrigen, gesättigten oder ungesättigten Heterocyclus mit bis zu 4 Heteroatomen aus der Reihe S, N oder O steht, die gegebenenfalls bis zu 3-fach gleich oder verschieden durch Halogen, Hydroxy, Nitro, Cyano, Trifluormethyl, Trifluormethoxy oder durch geradkettiges der verzweigtes Alkyl oder Alkoxy mit jeweils bis zu 8 Kohlenstoffatomen oder durch eine Gruppe der Formel $-NR^8R^9$ substituiert sind,

worin

$R^8$ und $R^9$ gleich oder verschieden sind und Wasserstoff, geradkettiges oder verzweigtes Alkyl mit bis zu 6 Kohlenstoffatomen oder Phenyl bedeuten,

$R^4$ für Wasserstoff, geradkettiges oder verzweigtes Alkyl mit bis zu 8 Kohlenstoffatomen oder Phenyl steht,

$R^5$ und $R^6$ gleich oder verschieden sind und
für Wasserstoff, Halogen, Cyano, Nitro, Trifluormethyl, Hydroxy, Trifluormethoxy oder für geradkettiges oder verzweigtes Alkyl oder Alkoxy mit jeweils bis zu 6 Kohlenstoffatomen stehen,

$R^7$ für Wasserstoff oder für geradkettiges oder verzweigtes Alkyl mit bis zu 6 Kohlenstoffatomen steht

und deren Salze.

**2.** Substituierte Imidazolyl-propensäurederivate nach Anspruch 1,

wobei

$R^1$ für geradkettiges oder verzweigtes Alkyl oder Alkenyl mit jeweils bis zu 6 Kohlenstoffatomen steht, die gegebenenfalls durch Cyclopropyl, Cyclobutyl, Cyclopentyl oder Cyclohexyl substituiert sind, für Cyclopropyl, Cyclopentyl oder Cyclohexyl steht,

$R^2$ für Wasserstoff, Fluor, Chlor, Brom, Iod, Trifluormethyl, Trifluormethoxy, Pentafluorethyl, Phenyl oder für geradkettiges oder verzweigtes Alkyl mit bis zu 6 Kohlenstoffatomen steht,

$R^3$ für Wasserstoff oder für geradkettiges oder verzweigtes Alkyl mit bis zu 8 Kohlenstoffatomen steht, oder

für Phenyl, Furyl, Thienyl, Imidazolyl, Pyrryl oder Pyridyl steht, die gegebenenfalls bis zu 2-fach gleich oder verschieden durch Fluor, Chlor, Brom, Hydroxy, Nitro, Cyano, Trifluormethyl, Trifluormethoxy oder durch geradkettiges oder verzweigtes Alkyl oder Alkoxy mit jeweils bis zu 6 Kohlenstoffatomen oder durch eien Gruppe der Formel $-NR^8R^9$ substituiert sind,

worin

$R^8$ und $R^9$ gleich oder verschieden sind und Wasserstoff oder geradkettiges oder verzweigtes Alkyl mit bis zu 4 Kohlenstoffatomen bedeuten,

$R^4$ für Wasserstoff oder für geradkettiges oder verzweigtes Alkyl mit bis zu 6 Kohlenstoffatomen steht,

$R^5$ und $R^6$ gleich oder verschieden sind und für Wasserstoff, Fluor, Chlor, Brom, Trifluormethyl, Trifluormethoxy oder für geradkettiges oder verzweigtes Alkyl mit bis zu 4 Kohlenstoffatomen stehen,

$R^7$ für Wasserstoff oder für geradkettiges oder verzweigtes Alkyl mit bis zu 4 Kohlenstoffatomen steht

und deren Salze.

**3.** Substituierte Imidazolyl-propensäurederivate nach Anspruch 1,

wobei

$R^1$ für geradkettiges oder verzweigtes Alkyl oder Alkenyl mit jeweils bis zu 4 Kohlenstoffatomen oder Cyclopropyl steht,

$R^2$ für Wasserstoff, Fluor, Chlor, Brom, Iod, Trifluormethyl, Pentafluorethyl, Phenyl oder für geradkettiges oder verzweigtes Alkyl mit bis zu 4 Kohlenstoffatomen steht,

$R^3$ für Wasserstoff oder geradkettiges oder verzweigtes Alkyl mit bis zu 6 Kohlenstoffatomen steht, oder

für Phenyl, Furyl oder Thienyl steht, die gegebenenfalls durch Fluor, Chlor, Cyano, Hydroxy, Trifluormethyl, Trifluormethoxy oder durch geradkettiges oder verzweigtes Alkyl oder Alkoxy mit jeweils bis zu 4 Kohlenstoffatomen oder durch eine Gruppe der Formel $-NR^8R^9$ substituiert sind,

worin

$R^8$ und $R^9$ gleich oder verschieden sind und Wasserstoff, Methyl oder Ethyl bedeuten,

$R^4$ für Wasserstoff oder für geradkettiges oder verzweigtes Alkyl mit bis zu 4 Kohlenstoffatomen steht,

$R^5$ und $R^6$ gleich oder verschieden sind und für Wasserstoff, Fluor, Chlor oder Brom stehen,

$R^7$ für Wasserstoff, Methyl, Ethyl, Propyl oder Isopropyl steht

und deren Salze.

**4.** Substituierte Imidazolyl-propensäurederivate nach Anspruch 1,

wobei

$R^1$ für geradkettiges oder verzweigtes Alkyl oder Alkenyl mit jeweils bis zu 4 Kohlenstoffatomen steht,

$R^2$ für Wasserstoff, Fluor, Chlor, Iod, Trifluormethyl oder Pentafluorethyl steht,

$R^3$ für Wasserstoff oder geradkettiges oder verzweigtes Alkyl mit bis zu 4 Kohlenstoffatomen steht, oder

für Phenyl, Furyl oder Thienyl steht, die gegebenenfalls durch Fluor, Chlor, Cyano oder Methoxy substituiert sind,

$R^4$ für Wasserstoff, Methyl oder Ethyl steht

und

$R^5$, $R^6$ und $R^7$ für Wasserstoff stehen

und deren Salze.

**5.** Substituierte Imidazolyl-propensäurederivate nach Anspruch zur Bekämpfung von Krankheiten.

**6.** Verfahren zur Herstellung von substituierten Imidazolyl-propensäurederivaten der allgemeinen Formel

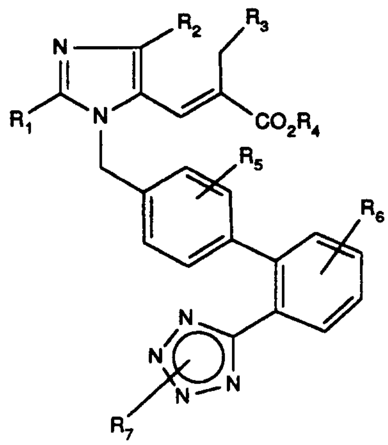

(I)

in welcher

$R^1$ für geradkettiges oder verzweigtes Alkyl oder Alkenyl mit jeweils bis zu 8 Kohlenstoffatomen steht, die gegebenenfalls durch Cycloalkyl mit 3 bis 6 Kohlenstoffatomen substituiert sind, für Cycloalkyl mit 3 bis 8 Kohlenstoffatomen steht,

$R^2$ für Wasserstoff, Halogen, Hydroxy, Nitro, Cyano, Trifluormethyl, Trifluormethoxy oder für Pentafluorethyl steht, oder für geradkettiges oder verzweigtes Alkyl mit bis zu 6 Kohlenstoffatomen steht, oder für Aryl mit 6 bis 10 Kohlenstoffatomen steht,

$R^3$ für Wasserstoff oder für geradkettiges oder verzweigtes Alkyl mit bis zu 10 Kohlenstoffatomen steht, oder für Aryl mit 6 bis 10 Kohlenstoffatomen oder für einen 5- bis 7-gliedrigen, gesättigten oder ungesättigten Heterocyclus mit bis zu 4 Heteroatomen aus der Reihe S, N oder O steht, die gegebenenfalls bis zu 3-fach gleich oder verschieden durch Halogen, Hydroxy, Nitro, Cyano, Trifluormethyl, Trifluormethoxy oder durch geradkettiges der verzweigtes Alkyl oder Alkoxy mit jeweils bis zu 8 Kohlenstoffatomen oder durch eine Gruppe der Formel $-NR^8R^9$ substituiert sind,

worin

$R^8$ und $R^9$ gleich oder verschieden sind und Wasserstoff, geradkettiges oder verzweigtes Alkyl mit bis zu 6 Kohlenstoffatomen oder Phenyl bedeuten,

$R^4$ für Wasserstoff, geradkettiges oder verzweigtes Alkyl mit bis zu 8 Kohlenstoffatomen oder Phenyl steht,

$R^5$ und $R^6$ gleich oder verschieden sind und für Wasserstoff, Halogen, Cyano, Nitro, Trifluormethyl, Hydroxy, Trifluormethoxy oder für geradkettiges oder verzweigtes Alkyl oder Alkoxy mit jeweils bis zu 6 Kohlenstoffatomen stehen,

$R^7$ für Wasserstoff oder für geradkettiges oder verzweigtes Alkyl mit bis zu 6 Kohlenstoffatomen steht

und deren Salze.

dadurch gekennzeichnet, daß man

Aldehyde der allgemeinen Formel (II)

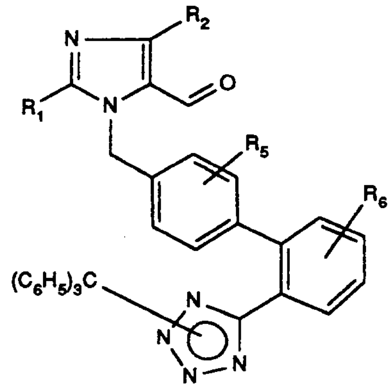

(II)

in welcher
$R^1$, $R^2$, $R^5$ und $R^6$ die oben angegebene Bedeutung haben,
zunächst durch Umsetzung mit Verbindungen der allgemeinen Formel (III)

$R^3-(CH_2)_2-CO_2R^{10}$ (III)

in welcher
$R^3$ die oben angegebene Bedeutung hat
und
$R^{10}$ die oben angegebene Bedeutung von $R^4$ hat aber nicht für Wasserstoff steht,
in inerten Lösemitteln, in Anwesenheit einer Base in die Verbindungen der allgemeinen Formel (IV)

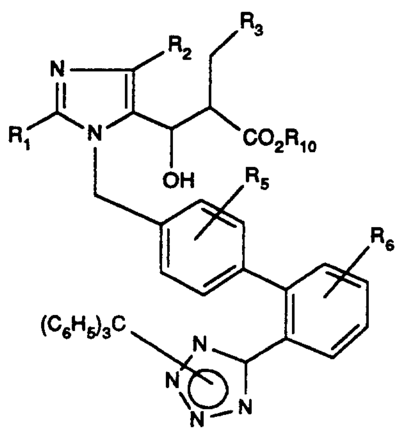

(IV)

in welcher
$R^1$, $R^2$, $R^3$, $R^5$, $R^6$ und $R^{10}$ die oben angegebene Bedeutung haben,
überführt

anschließend die freie Hydroxyfunktion durch Einführung einer Schutzgruppe blockiert und in einem letzten Schritt eine Eliminierung in inerten Lösemitteln in Anwesenheit einer Base durchführt,

und im Fall der Säuren ($R^4$ = H) die Ester verseift,

und im Fall daß $R^7$ nicht für Wasserstoff steht, die −NH−Funktion alkyliert.

**7.** Verfahren nach Anspruch 6, dadurch gekennzeichnet, daß man die Umsetzung in einem Temperaturbereich von − 100 °C bis + 100 °C durchführt.

**8.** Arzneimittel enthaltend mindestens ein substituiertes Imidazolyl-propensäurederivate nach Anspruch 1.

9. Arzneimittel nach Anspruch 8 zur Behandlung von Bluthochdruck und Atherosklerose.

10. Verwendung von substituierten Imidazolyl-propensäurederivaten nach Anspruch 1 zur Herstellung von Arzneimitteln.

Europäisches Patentamt

# EUROPÄISCHER TEILRECHERCHENBERICHT

der nach Regel 45 des Europäischen Patentübereinkommens für das weitere Verfahren als europäischer Recherchenbericht gilt

Nummer der Anmeldung

EP 92 11 5969

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int. Cl. 5) |
|---|---|---|---|
| D,A | EP-A-0 403 158 (SMITH BEECHAM CORPORATION)<br>--- | | C07D403/10<br>A61K31/415<br>C07D409/14<br>A61K31/41<br>A61K31/38 |
| D,A | EP-A-0 403 159 (SMITH BEECHAM CORPORATION)<br>----- | | |

| RECHERCHIERTE SACHGEBIETE (Int. Cl. 5) |
|---|
| C07D<br>A61K |

## UNVOLLSTÄNDIGE RECHERCHE

Nach Auffassung der Recherchenabteilung entspricht die vorliegende europäische Patentanmeldung den Vorschriften des Europäischen patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche:

Unvollständig recherchierte Patentansprüche:

Nicht recherchierte Patentansprüche:

Grund für die Beschränkung der Recherche:

Siehe Ergänzungsblatt C

| Recherchenort | Abschlußdatum der Recherche | Prüfer |
|---|---|---|
| DEN HAAG | 22 DEZEMBER 1992 | DE BUYSER I.A.F. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EP 92 11 5969 -C-

UNVOLLSTÄNDIG RECHERCHIERTE PATENTANSPRÜCHE : 1-10

Grund : Die Abfassung der Ansprüche ist nicht klar und knapp zu fassen (Art. 83-84, EPA) und enthalt eine so grosse Zahl Verbindungen dass eine vollständige Recherche auf ökonomischer Gründe nicht möglich ist (Siehe Richtlinien für die Prüfung im Europaïschen Patentamt, Teil B, Kapittel III.2 (Umfang der Recherche)).
Geleitet durch den Sinn der Anfrage und die erfinderische Idee als offenbart in die Beschreibung der vorliegende Anfrage, ist die Recherche gegründet auf die Beispiele.